# EUROPEAN PATENT APPLICATION

(11) **EP 3 819 380 A1**
(43) Date of publication of application: **12.05.2021**
(21) Application number: 19382964.5
(22) Date of filing: 05.11.2019
(51) Int. Cl.: C12P 7/62, C12R 1/40, C12N 1/20

(54) **RECOMBINANT PSEUDOMONAS PUTIDA STRAINS FOR THE PRODUCTION OF POLYHYDROXYALKANOATE**

(71) Applicant: Consejo Superior de Investigaciones Científicas (CSIC), 28006 Madrid (ES)
(72) Inventor: PRIETO JIMENEZ, Maria Auxiliadora, 28040 MADRID (ES); NOGALES ENRIQUE, Juan, 28049 MADRID (ES); MANOLI, María Tsampika, 28040 MADRID (ES)
(74) Representative: Pons

(57) **Abstract**

The invention relates to recombinant *Pseudomonas putida* strains derived from *P. putida* KT2440 which harbor genetic modifications that make them able to produce significant amounts of polyhydroxyalkanoate (PHA) under balanced nutritional conditions, even by using fatty acid-unrelated carbon sources. Genetic modifications comprised in these recombinant strains focus on avoiding nitrogen limitation and overproducing PHA. The invention also refers to a method for the production of PHA in which the strains of the invention are used.

SEQUENCE LISTING

<110>  Consejo Superior de Investigaciones Científicas (CSIC)

<120>  RECOMBINANT PSEUDOMONAS PUTIDA STRAINS FOR THE PRODUCTION OF
       POLYHYDROXYALKANOATE

<130>  EP1641.1500

<160>  13

<170>  PatentIn version 3.5

<210>  1
<211>  1680
<212>  DNA
<213>  Artificial Sequence

<220>
<223>  phaC1 gene

<400>  1
atgagtaaca agaacaacga tgagctacag cggcaggcct cggaaaacac cctggggctg      60

aacccggtca tcggcatccg ccgcaaggac ctgttgagct cggcacgcac cgtgctgcgc     120

caggccgtgc gccaaccgct gcacagcgcc aagcatgtgg ctcactttgg cctggagctg     180

aagaacgtgt tgctgggcaa atccagcctg gccccggaca gcgacgaccg tcgcttcaat     240

gacccggcct ggagcaacaa cccgctgtac cgccgctacc tgcaaaccta cctggcctgg     300

cgcaaggagc tgcaggactg ggtgagcagc agcgacctgt ccccccagga catcagccgc     360

ggccagttcg tcatcaacct gatgaccgag gccatggcgc cgaccaatac cctgtccaac     420

ccggctgcgg tcaaacgctt cttcgaaacc ggcggcaaga gcctgctcga tggcctgtcc     480

aacctggcca aggacatggt caacaacggc ggcatgccca gccaggtgaa catggatgcc     540

ttcgaagtgg gcaagaacct gggcaccagc gaaggcgcgg tggtgtaccg caacgatgtg     600

ctggaactga tccagtacag ccccatcacc gagcaggtgc atgcccgtcc gctgctggtg     660

gtgccaccgc agatcaacaa gttctacgtg ttcgacctca gcccggaaaa gagcctggcg     720

cgcttctgcc tgcgctcgca gcagcagacc ttcatcatca gctggcgcaa cccgaccaag     780

gcccagcgtg aatggggcct gtccacctac atcgatgcgc tgaaagaagc cgtcgacgcg     840

gtgctgtcga ttaccggcag caaggacctg aacatgctcg gcgcctgctc cggtggcatc     900

acttgtaccg cactggtggg ccactatgcc gccattggcg agaacaaggt caacgccctg     960

accctgctgg tcagcgtgct ggacaccacc atggacaacc aggttgcttt gtttgtcgac    1020

gagcagacct tggaggccgc caagcgccac tcctatcagg cgggcgtgct ggaaggcagc    1080

gaaatggcca aggtgttcgc ctggatgcgc cccaacgacc tgatctggaa ctactgggta    1140

aacaactacc tgctcggcaa tgagcccccc gtgttcgaca tcctgttctg gaacaacgac    1200

accacgcgcc tgccggccgc cttccacggc gacctgatcg aaatgttcaa gagcaacccg    1260

ctgacccgcc ccgacgccct ggaagtgtgc ggcaccgcga tcgacctgaa acaggtcaaa 1320

tgcgacatct acagcctcgc cggcaccaac gaccacatca ccccctggcc gtcatgctac 1380

cgctcggcac atctgttcgg cggcaagatc gaattcgtac tgtccaacag cgggcatatc 1440

cagagcatcc tcaacccgcc gggcaacccg aaggcacgtt tcatgaccgg tgccgatcgc 1500

ccgggtgacc cggtggcctg gcaggaaaat gccatcaagc atgcagactc ctggtggttg 1560

cactggcaga gttggctggg cgagcgtgcc ggcgcgctga aaaaggcacc gacccgcctg 1620

ggcaaccgta cctatgccgc cggcgaagcc tccccaggca cctacgttca cgagcgttga 1680

<210> 2
<211> 786
<212> DNA
<213> Artificial Sequence

<220>
<223> phaF gene

<400> 2
atggctggca agaagaacac cgaaaaagaa ggcagctcct gggtcggcgg gatcgagaaa 60

tactcccgca agatctggct ggcggggctg ggtatctatt cgaagatcga ccaggacggc 120

ccgaagctgt tcgactcgct ggtgaaggat ggcgagaagg ccgagaagca ggcgaaaaag 180

acggctgaag atgttgccga gactgccaag tcatcgacca cttcgcgggt gtcgggcgtg 240

aaggaccgtg cgctgggcaa gtggagcgaa cttgaagaag ccttcgacaa gcgccttaac 300

agcgccatct cgcgccttgg cgtgccgagc cgcaacgaga tcaaggcact gcaccagcag 360

gtggacagcc tgaccaagca gatcgagaag ctgaccggtg cttcggttac gccgatttcg 420

tcgcgcgctg cagcaaccaa gccggctgca agcaaggctg cggccaagcc actggccaag 480

gcagcagcta agcctgcggc gaaaacggcg gcggccaaac ctgctggcaa aaccgcagcg 540

gccaaacccg cagccaaaac cgcagcggaa aaacctgcag ctaagccagc agccaagcct 600

gcagcggcca aacctgcggc agccaagaaa cctgcggtga agaaagctcc agccaaaccg 660

gcagcggcca aaccagcagc accagctgcc agcgctgcgc ctgcagcgac cacagcaccg 720

gcaactgccg ccaccccggc cagcagcacg ccgtcggcac cgactggcac cggtaccctg 780

atctga 786

<210> 3
<211> 888
<212> DNA
<213> Artificial Sequence

<220>
<223> phaG gene

<400> 3

atgaggccag aaatcgctgt acttgatatc caaggtcagt atcgggttta cacggagttc 60

tatcgcgcgg atgcggccga aaacacgatc atcctgatca acggctcgct ggccaccacg 120

gcctcgttcg cccagacggt acgtaacctg cacccacagt tcaacgtggt tctgttcgac 180

cagccgtatt caggcaagtc caagccgcac aaccgtcagg aacggctgat cagcaaggaa 240

accgaggcgc atatcctcct tgagctgatc gagcacttcc aggcagacca cgtgatgtct 300

ttttcgtggg gtggcgcaag cacgctgctg gcgctggcgc accagccgcg gtacgtgaag 360

aaggcagtgg tgagttcgtt ctcgccagtg atcaacgagc cgatgcgcga ctatctggac 420

cgtggctgcc agtacctggc cgcctgcgac cgttatcagg tcggcaacct ggtcaatgac 480

accatcggca agcacttgcc gtcgctgttc aaacgcttca actaccgcca tgtgagcagc 540

ctggacagcc acgagtacgc acagatgcac ttccacatca accaggtgct ggagcacgac 600

ctggaacgtg cgctgcaagg cgcgcgcaat atcaacatcc cggtgctgtt catcaacggc 660

gagcgcgacg agtacaccac agtcgaggat gcgcggcagt tcagcaagca tgtgggcaga 720

agccagttca gcgtgatccg cgatgcgggc cacttcctgg acatggagaa caagaccgcc 780

tgcgagaaca cccgcaatgt catgctgggc ttcctcaagc caaccgtgcg tgaaccccgc 840

caacgttacc aacccgtgca gcaggggcag catgcatttg ccatctga 888

<210> 4
<211> 1683
<212> DNA
<213> Artificial Sequence

<220>
<223> PP_0763 gene

<400> 4
atgttgcaga cacgcatcat caagcccgcc gagggcgcct atgcctatcc attgctgatc 60

aagcgcctgc tgatgtccgg cagccgctat gaaaagaccc gggaaatcgt ctaccgcgac 120

cagatgcggc tgacgtatcc acagctcaac gagcgcattg cccgcctggc caacgtgctg 180

accgaggccg gggtcaaggc cggtgacacc gtggcggtga tggactggga cagccatcgc 240

tacctggaat gcatgttcgc catcccgatg atcggcgctg tggtgcacac catcaacgtg 300

cgcctgtcgc ccgagcagat cctctacacc atgaaccatg ccgaggaccg cgtggtgctg 360

gtcaacagcg acttcgtcgg cctgtaccag gccatcgccg ggcagctgac cactgtcgac 420

aagaccctgc tactgaccga tggcccggac aagactgccg aactgcccgg tctggtcggc 480

gagtatgagc agctgctggc tgctgccagc ccgcgctacg acttcccgga tttcgacgag 540

aattcggtgg ccactacctt ctacaccact ggcaccaccg gtaaccccaa gggcgtgtat 600

ttcagtcacc gccagctggt gctgcacacc ctggccgagg cctcggtcac cggcagtatc 660

gacagcgtgc gcctgctggg cagcaacgat gtgtacatgc ccatcacccc gatgttccac 720

gtgcatgcct ggggcatccc ctacgctgcc accatgctcg gcatgaagca ggtgtaccca 780

gggcgctacg agccggacat gctggtcaag ctttggcgtg aagagaaggt cactttctcc 840

cactgcgtgc cgaccatcct gcagatgctg ctcaactgcc cgaacgccca ggggcaggac 900

ttcggcggct ggaagatcat catcggcggc agctcgctca accgttcgct gtaccaggcc 960

gccctggcgc gcggcatcca gctgaccgcc gcgtatggca tgtcggaaac ctgcccgctg 1020

atctccgcgg cacacctgaa cgatgaactg caggccggca gcgaggatga gcgcgtcact 1080

taccgtatca aggccggtgt gccggtgccg ttggtcgaag cggccatcgt cgacggcgaa 1140

ggcaacttcc tgcccgccga tggtgaaacc cagggcgagc tggtactgcg tgcgccgtgg 1200

ctgaccatgg gctacttcaa ggagccggag aagagcgagg agctgtggca gggcggctgg 1260

ctgcacaccg gtgacgtcgc caccctcgac ggcatgggct acatcgacat ccgcgaccgc 1320

atcaaggatg tgatcaagac cggtggcgag tgggtttcct cgctcgacct ggaggacctg 1380

atcagccgcc acccggccgt gcgcgaagtg gcggtggtgg gggtggccga cccgcagtgg 1440

ggtgagcgcc cgtttgccct gctggtggca cgtgacggcc acgatatcga cgccaaggcg 1500

ctgaaggaac acctcaagcc attcgtcgag caaggtcata tcaacaagtg ggcgattcca 1560

agccagatcg cccttgttac tgaaattccc aagaccagtg tcggcaagct cgacaagaaa 1620

cgcattcgcc aggacatcgt ccagtggcag gccagcaaca gcgcgttcct ttccacgttg 1680

taa 1683

<210> 5
<211> 1641
<212> DNA
<213> Artificial Sequence

<220>
<223> aceF gene

<400> 5
atgagcgaac tcattcgcgt acctgacatc ggcagcggtg aaggtgaaat catcgagctg 60

ttcgtcaagg tcggtgaccg tatcgaggct gaccagagcc tgctgaccct ggagtccgac 120

aaggcctcca tggaaattcc ggcgcccaag gctggcgtga tcaaggaact gaaggtcaag 180

ctgggcgacc gcctgaaaga aggcgacgaa ctgctggtcc tggaagccga gggcgccgct 240

gctgcggccc ctgaggcgcc ggctgctgcc gctgctccgg ctgcggcgcc agcgccagcc 300

gctgaagccg ctcctgctgc tccggccgca gccccggctg ctgccagcgt gcaggacatc 360

cacgtgccgg acatcggttc gtcgggcaag gccaagatca tcgaagtgct ggtcaaagtc 420

ggcgacaccg tcgaagccga ccagtcgctg attaccctgg agtccgacaa ggcctccatg 480

gaaatcccgt cgccggctgc cggcgtggtc gaagaagtgc tgtgcaagct ggaggacgaa 540

gtcggcactg gcgacctgat cttcaagctg aaagtggcgg gtgccgcccc ggctgccgcg 600

ccggcaccgg ctgctgctcc tgcaccagcc aaggctgagg ctgctccggc tgccgcacct 660

gccgctgccg ccccggctgc tgcccctgca ccggctgcta ccgcaccggc cgctggcagc 720

aatgccaagg ttcacgcagg ccccgctgtt cgtcagctgg cccgtgaatt cggtgtcgag 780

ctgggtgcag ttgctgccac cggcccgcac ggtcgcatcc tgaaagagga cgtgcaggtc 840

tacgtcaagg ccatgatgca gaaggccaag gaagcgccgg cagccgccgg tgcgaccggt 900

ggtgctggca tcccgccgat ccctgccgtg gacttcagca agttcggtga agtggaagaa 960

gtggccctga cccgcctgat gcaggtcggt gctgccaacc tgcaccgcag ctggctcaac 1020

gtgccgcacg tgacccagtt cgactctgcc gacatcaccg agctggaagc cttccgcgtt 1080

gcgcagaagg ccgtggccga gaaggctggg gtgaagctga ccgtgctgcc actgctgctc 1140

aaggcctgcg ccttcctgct caaggaactg ccggacttca acagctcgct ggcgccaagc 1200

ggcaaggcca tcatccgcaa gaagtacgtg cacatcggct tcgccgtgga caccccggac 1260

ggcctgctgg tccctgtgat caagaacgtc gaccagaaga gcctgctgca actggctgcc 1320

gaagccgctg cactggccga gaaggcgcgc accaagaagc tgtcggctga cgagatgcaa 1380

ggcgcctgct tcaccatctc cagcctcggc cacattggcg gcaccggctt cacgccgatc 1440

gtcaatgcgc cggaggtggc tatcctgggc gtctccaagg cgaccatgca gccagtctgg 1500

gatggcaagg ccttccagcc aaagctgatg ttgccgctgt cgctgtccta cgatcaccgt 1560

gtgatcaacg gcgctgccgc cgcgcgcttc accaagcgcc tgggcgacgt gctgggcgat 1620

atccgcacca tgctgctgta a 1641

<210> 6
<211> 2646
<212> DNA
<213> Artificial Sequence

<220>
<223> aceE gene

<400> 6
atgcaagacc tcgatccaat cgaaacccag gaatggctgg atgccctgga gtcggtcctc 60

gacaaagaag gcgaggaccg cgctcattac ctgatgaccc gtatgggcga gctggccacc 120

cgtagtggct cccagctgcc atatgcgatc accacgccat accgcaacac catccctgtc 180

acccacgaag cacgcatgcc tggcgacctg ttcatggaac gccgcattcg ctcgatggtg 240

cgttggaacg cgctggccat ggtcatgcgt accaacctga aagactcgga cctgggcgga 300

cacatctcta gcttcgcctc cagtgccacc ctgtacgaca tcggcttcaa ctacttcttc 360

caggccccta ccgaagaaca cggcggcgac ctgatcttct tccagggcca cgcatctcca 420

ggcgtctacg cccgtgcctt catggaaggc cgtatcagcg aggaccagat gaacaacttc 480

cgccaggaag tggacggcaa cggcctgtcg tcgtacccgc acccatggct gatgcctgac 540

ttctggcagt tcccgaccgt ttcgatgggt ctgggcccga tccaggcgat ctaccaggca 600

cgcttcatga agtacctgga agcccgtggc ttcatcccgg ccggcaagca gaaggtctgg 660

tgcttcatgg gcgacggtga gtgcgacgag ccggaatccc tgggtgcaat cgccctggcc 720

ggccgcgaga agctggacaa cctgatcttc gtcatcaact gcaacctgca gcgcctcgac 780

ggcccggttc gcggcaacgg caagatcatc caggaactcg aaggcgtgtt ccgtggcggt 840

ggctggaacg tcaacaaagt cgtctggggc cgtttctggg acccactgct ggccaaggac 900

accaacggtg ccctgcagcg ccgcatggac gaagtcatcg acggcgaata ccagaactac 960

aaagccaaag acggcgcgta cgttcgcgag cacttcttca acaccccgga actcaaggcc 1020

atggtcgagg acctgtccga cgaagagatc tggaagctca accgtggcgg tcacgacccg 1080

tacaaggttt acgcggccta tcaccaggct gtgaaccaca aagagcagcc gaccgtcatc 1140

ctggccaaga ccatcaaggg ttacggcacc ggtgctggcg aagccaagaa caccgcgcac 1200

aacaccaaga aggtcgacgt cgacagcctg cgtcacttcc gtgaccgctt cgacatcccg 1260

gtcaaggatg ccgacctgga gaacctgccg ttcttcaagc cggaagaagg ttctgccgaa 1320

gccaagtacc tggccgagcg ccgcgcagcc ctgggtggct tcgtaccgca gcgccgtgcc 1380

aagagcttca gcgtgccgac cccgccactg gaaacgctga aagcgatcct ggacggctcg 1440

ggcgaccgcg aaatctccac caccatggcc ttcgtgcgta ttctggcgca gctggtcaag 1500

gacaaggaca ttggccagcg catcgtcccg atcatcccgg acgaagcccg taccttcggt 1560

atggaaggca tgttccgcca gttgggcatc tactcgtcgg tcggccagct ctacgagcca 1620

gtcgataaag accaggtgat gttctaccgc gaggacaaga agggccagat cctcgaggaa 1680

ggcatcaacg aagccggcgc catgtcgtcg ttcatcgctg ccggtacctc gtacagctgc 1740

cacaaccagc cgatgctgcc gttctacatc ttctactcga tgttcggttt ccagcgtatt 1800

ggcgacctgg cctgggccgc tggcgacagc cgcacccgtg gtttcctgat cggcggtacc 1860

gccggccgta ccaccctcaa cggtgaaggc ctgcagcacg aggacggtca cagccacatg 1920

atggcgggca ccatcccgaa ctgccgcacc tacgatccga cctacggcta cgagctggcg 1980

gtgatcatcc aggacggcat gaagaagatg accgaagagc aacaggacat cttctactac 2040

atcaccgtga tgaacgaatc ctaccagcag ccagccatgc cggccggtgt cgaggaaggc 2100

atcatcaagg gcatgtacct gctggaagag gacacccgcg aagccgcaca ccacgtacag 2160

ctgatgggct ccggcaccat cctgcgcgaa gtccgcgaag ccgcgaagat cctgcgtgaa 2220

gagttcaacg tcggtgccga cgtgtggagc gtcaccagct tcaacgaact gcgtcgcgac 2280

ggcctggccg tggaacgcgc caaccgcctg aagcctggcc agaagccaca gcagacctac 2340

gtcgagcagt gcctgaacgg tcgcaaaggc ccggtcatcg cctctaccga ctacatgaag 2400

ctgttcgccg agcagattcg tcagtgggta ccgagcaaag agttcaaagt cctgggtacc 2460

gacggttacg gtcgcagcga cagccgcaag aagctgcgtc acttcttcga agtcgaccgc 2520

cacttcgtgg tgctggctgc cctggaagcc ctggctgacc gtggcgagat cgaacccaag 2580

gttgttgcag acgctatcgt caagttcggt atcgacccgg acaagcgcaa cccactggac 2640

tgctga 2646

<210> 7
<211> 1437
<212> DNA
<213> Artificial Sequence

<220>
<223> lpdG gene

<400> 7
atgacccaga aattcgacgt agtggtaatc ggtgcaggcc caggcggcta tgtggctgcc 60

atcaaggctg cccaacttgg tctgaaaact gcctgtatcg agaagtacac cgacgccgag 120

ggcaaactgg ccctgggcgg cacctgcctg aacgtaggtt gcattccttc caaggcgctg 180

ctggacagct cctggaagta caaggaagcc aaagagagct tcaacgtcca cggtatctcc 240

acgggcgaag tgaagatgga cgtcgccgcg atggttggcc gcaaggccgg catcgtcaag 300

aacctgaccg gtggcgttgc caccctgttc aaggccaacg gcgtcacttc gatccagggc 360

cacggcaagc tgctggccgg caagaaagtc gaagtcacca aggctgacgg caccaccgaa 420

gtcatcgaag ccgagaacgt gatcctggct tccggctcgc gcccgatcga cattccgccg 480

gccccggtcg accagaacgt catcgtcgac tccaccggcg ccctggaatt ccaggccgta 540

ccgaagcgcc tgggcgttat cggtgcaggc gtgatcggcc tggagctggg ttcggtatgg 600

gctcgcctgg gtgctgaagt gaccgtcctg gaagccctgg acaccttcct gatggctgcc 660

gacaccgccg tgtcgaaaga agcccagaaa accctgacca agcaaggcct ggacatcaag 720

ctgggcgctc gcgtcaccgg ctcgaaagtc aacggcaatg aagtcgaagt gacctacacc 780

aacgccgaag gcgagcagaa gatcaccttc gacaagctga ttgttgcagt cggccgtcgc 840

ccggtgacca ccgatctgct ggcttccgac agcggcgtga ccatcgacga gcgtggctac 900

atcttcgtcg acgattactg cgccaccagc gttccgggcg tgtacgccat cggtgacgtg 960

gtacgcggta tgatgctggc ccacaaggct tcggaagagg gcatcatggt cgtcgagcgc 1020

atcaagggcc acaaggccca gatgaactac gacctgatcc cgtcggtcat ctacacccac 1080

ccggaaatcg cgtgggtcgg caagaccgaa caggccttga aggccgaggg cgttgaggtt 1140

aacgtgggca ccttcccgtt cgcggccagc ggccgtgcga tggctgccaa cgacaccggt 1200

ggtttcgtca aggtcatcgc cgatgccaag accgaccgcg ttctgggtgt tcacgtgatt 1260

ggcccatcgg ctgccgaact ggtgcagcag ggcgcaatcg caatggaatt cggcaccagt 1320

gccgaggatc tgggcatgat ggtgttcagc catccaaccc tgtccgaagc gttgcatgaa 1380

gcagcgctgg cggtgaatgg cggcgccatt cacgtggcca accgtaagaa gcgttaa 1437

<210> 8
<211> 103
<212> DNA
<213> Artificial Sequence

<220>
<223> LambdaT0 terminator

<400> 8
   cttggactcc tgttgataga tccagtaatg acctcagaac tccatctgga tttgttcaga 60
   acgctcggtt gccgccgggc gttttttatt ggtgagaatc cag 103

<210> 9
<211> 41
<212> DNA
<213> Artificial Sequence

<220>
<223> SynPro16 promoter

<400> 9
ggtaccttga caaagcgctt acctctttct ataatataga g 41

<210> 10
<211> 6
<212> DNA
<213> Artificial Sequence

<220>
<223> standard RBS sequence

<400> 10
aggggg 6

<210> 11
<211> 105
<212> DNA
<213> Artificial Sequence

<220>
<223> LambdaT1 terminator

<400> 11
tctagggcgg cggatttgtc ctactcagga gagcgttcac cgacaaacaa cagataaaac 60

gaaaggccca gtctttcgac tgagcctttc gttttatttg atgcc 105

<210> 12
<211> 82
<212> DNA

<213>  Artificial Sequence

<220>
<223>  rnpB-T1 terminator

<400>  12
tcggtcagtt tcacctgatt tacgtaaaaa cccgcttcgg cgggtttttg cttttggagg      60

ggcagaaaga tgaatgactg tc                                              82


<210>  13
<211>  83
<212>  DNA
<213>  Artificial Sequence

<220>
<223>  rpoC-term terminator

<400>  13
gtaatcgtta atccgcaaat aacgtaaaaa cccgcttcgg cgggtttttt tatgggggga      60

gtttagggaa agagcatttg tca                                             83

## Description

The invention belongs to the field of industrial biotechnological processes for the production of polyhydroxyalkanoates (PHA) by recombinant microorganisms, and more particularly to genetically engineered *Pseudomonas putida* strains that overproduce PHA.

### BACKGROUND ART

Oil-based synthetic polymers, such as plastics, are becoming a serious environmental problem nowadays. Often, they are burned increasing carbon dioxide emissions and producing harmful dioxins and endocrine disruptors. In other cases, plastics massive production and their inappropriate use during the last decades, led to plastic pollution of lands, seas, oceans etc., creating environmental worries. In fact, in 2017, the global plastic pollution reached up to almost 350 million tons with 64.4 million tons only produced in Europe (data obtained from market studies provided by PlasticsEurope (PEMRG) and converted Market and Strategy GmbH). Polyesters produced by microorganisms, such as polyhydroxyalkanoates (PHA), can be incorporated into biodegradable natural recycling system and are seemed as promising alternative to oil-based plastics. For this reason, a large effort has been made in order to optimize the production of PHA by using biochemical engineering approaches.

PHA are accumulated in microorganisms as energy and carbon source under unbalanced nutritional conditions, which mainly include an excess of carbon together with the lack of other nutrients such as phosphorus, nitrogen and oxygen. Subsequently, PHA production strategies involve processes based on nutrient limitation where PHA accumulation mainly occurs at the offset of the exponential phase growth in the fed-batch strategies or in a continuous process under single or double limitation conditions. However, there are several drawbacks associated to these nutrient limitation regimes. The main problem derived from this is the low yield of total biomass in the process, which is very important when considering that the product location is the cytoplasm of the bacterial cells. Although recombinant microorganisms have been thoroughly used for improving yields, the requirement of nutrient unbalance in the process has not been overcome. All these important limitations largely hamper the cost-effective production of PHA.

Despite PHA have been commercially developed and marketed, their production costs are much higher than those of conventional petrochemical-based plastics, because of the low production yields of bacteria, the need of high cell density cultures due to the intracellular location of the product, and downstream processes.

Concerning the feedstock, PHA have been produced by using wastes containing different carbon sources, being the fatty acids the best precursors in the case of *Pseudomonas.* Fatty acid-unrelated carbon sources, such as carbohydrates or glycerol-containing residues, have been also used, however the yields are significantly lower than those achieved with fatty acid wastes.

Nowadays, there is an increasing interest in the revalorization of complex and recalcitrant polymers including lignin and oil-based plastics, such as polyethylene terephthalate (PET) and polyurethane (PU). Many of these heterogeneous substrates are degraded via the central intermediate protocatechuic acid (PCA) through the β-ketoadipate pathway. Monomers derived from the hydrolysis of these polymers have an intrinsic value, however the complete revalorization of these compounds require complex and expensive purification steps.

Among the main challenges hampering the optimal production of PHA stand out: i) the mandatory nitrogen limitation required to achieve a significant PHA production, which largely limits the amount of bacterial biomass in the bioreactor, and ii) the low carbon flux allocated to synthetize PHA using unrelated carbon sources, which results in negligible PHA production even under nitrogen limitation.

*Pseudomonas putida* KT2440 strain is a bacterium that produces PHA and possesses excellent features that enable it to be used in large-scale bioplastic production processes. Besides its huge metabolic diversity, it is certified by the American Food and Drug Administration (FDA) as HV1 (Host vector system safety level 1) strain, indicating it is safe to use in a P1 or ML1 environment, but it cannot be used as food additive. It is known that *P. putida* KT2440 is capable of producing PHA from a variety of carbon sources, such as glucose, fructose, glycerol, octanoate, succinate, aromatic compounds, among others (Escapa, I.F., et al., 2013, Environ. Microbiol., 15(1): 93-110; Poblete-Castro, I., et al., 2013, Metab. Eng., 15: 113-123; Fonseca, P., et al., 2014, Int. J. Biol. Macromol., 71: 14-20).

The genes involved in the metabolism of PHA synthesis in *P. putida* have been identified. This information has been proposed for constructing transgenic strains able to utilize carbon sources more efficiently and better produce PHAs (Wang Q, et. al., 2010, J Biosci Bioeng., 110(6):653-9; Poblete-Castro, I., et al., 2013, Metab. Eng., 15: 113-123; Salvachúa, D., et al., 2019, Microb. Biotechnol., DOI: 10.1111/1751-7915.13481). Recently, recombinant *P. putida* strains have been obtained, able to produce high yields of PHA from aromatic compounds and lignin. In fact, these strains lacked the *phaZ* and the *fadBA* genes involved in PHA depolymerization and β-oxidation pathway, respectively. To increase the carbon flux towards PHA production, key enzymes involved in this process, such as *phaC1* and *phaC2* synthases, *phaG* and PP_0763, have been overexpressed (Salvachúa, D., et al., 2019, Microb. Biotechnol., DOI: 10.1111/1751-7915.13481).

WO2011/086211 describes a *P. putida* KT2440 strain genetically modified at *Tol-pal* genes in order to improve the PHA extraction.

The development of a method for facilitating the production of PHAs in *P. putida* from glycerol has been also reported. For said purpose, a mutant strain derived from *P. putida* was designed which comprises a mutation in the *glpR* gene that enables the bacterium to use glycerol as a carbon source in a more efficient manner, and thus makes it possible to improve the production of biomass and PHA (WO2013072541).

Recombinant *P. putida* KT2440 strains have been also developed to reduce the substrate cost, which represents nearly 50% of the total PHA production cost. Thus, for instance, xylose, a hemicellulose derivate, has been tested as the growth carbon source in a recombinant *P. putida* KT2440 strain engineered to contain the genes encoding xylose isomerase (XylA) and xylulokinase (XylB from *Escherichia coli* W3110. Sequential feeding of relatively cheap carbohydrates and expensive fatty acids has been proposed in this approach as a practical way to achieve more cost-effective PHA production. However, this process based on this recombinant *P. putida* strain does not allow to achieve high cell densities and high productivity of PHA (Sylvaine Le Meur, et al., 2012, BMC Biotechnology, Vol. 12: 53).

Therefore, the design of improved microorganisms capable of producing large amounts of PHA from inexpensive carbon sources and in the absence of nutrients limitation, while guiding a high cell density, is still of critical importance.

### DESCRIPTION OF THE INVENTION

This invention solves the problem mentioned above by providing recombinant (engineered) *Pseudomonas putida* KT2440 strains capable of producing and accumulating significant amounts of PHA from unrelated, cheap and highly recalcitrant carbon sources, such as glucose and aromatic compounds derived from lignin and PET, in the absence of nutritional limitations, i. e. under balanced nutrient conditions.

Through several mutations in the starting *P. putida* KT2440 strain, this invention successfully achieves an optimal and improved production of PHA under balanced nutritional conditions, i. e. in the absence of nutrient (nitrogen) limitation, even by using fatty acid-unrelated carbon sources.

The use of inexpensive and abundant compounds, such as sugars, as feedstock for PHA production is interesting, since they can largely contribute to the cost-effectiveness of the biotechnological PHA production process.

Thus, the PHA production bioprocess supported by the strains of the invention constitutes a significant improvement with respect to current PHA production processes, since it allows to use cheaper and complex feedstocks while avoids nutrient limitation, thus promoting higher cell densities. All of these advantages contribute to a more effective PHA production, increasing the yield of the process.

The strains of the invention have been designed in order to avoid the nitrogen limitation required in traditional PHA production strategies. This is achieved by removing the natural regulation of PHA gene cluster and by building a synthetic operon responsible of constitutive expression of those genes required for PHA production. Furthermore, some of the mutations included in the strains of the invention are responsible of optimizing the carbon flux towards PHA biosynthesis.

Thus, one aspect of the present invention relates to a recombinant *Pseudomonas putida* KT2440 strain, hereinafter "the strain of the invention", characterized in that:
- its *pha* cluster is deleted (this genetic mutation will be also referred to in the present invention as *"Δpha"*), and
- it comprises a synthetic operon comprising the nucleotide sequences of the *phaC1* gene (preferably SEQ ID NO: 1), the *phaF* gene (preferably SEQ ID NO: 2), the *phaG* gene (preferably SEQ ID NO: 3) and the PP_0763 gene (preferably SEQ ID NO: 4).

*Pseudomonas* spp., is a continuously growing genus (approximately 300 species) of Gram negative, aerobic, bacillus, belonging to γ-proteobacteria class. *Pseudomonas putida* strain is a "paradigm of class of cosmopolitan bacteria", isolated from a variety of environments such as soils, plant rhizosphere and water. *P. putida* KT2440 strain derives from the toluene-degrading organism initially isolated in Japan and designated as *P*. *arvilla* mt-2 (mt-2, stands for "meta-toluate degrader, isolate 2") and later renamed to *P. putida* mt-2 (Williams, P.A et al., 1974, J. Bacteriol, 120(1): 416-23).

Since the publication and the recent revision of the *P. putida* KT2440 genome (Nelson, K.E., et al., 2002, Environ. Microbiol. 4(12):799-808; Belda, E., et al., 2016, Environ. Microbiol., 18(10): 3403-3424), much effort has been invested in exploiting its capacities in biotechnological applications. Furthermore, *P. putida* metabolism has been extensively characterized and seven genome-scale models (known as GEMs) are available *i*JN1411 (Nogales, J., et al., 2017, bioRxiv, doi: https://doi.org/10.1101/139121).

The "*pha* cluster" refers to the genes involved in PHA metabolism, these genes are *phaC1* (PP_5003), *phaZ* (PP_5004), *phaC2* (PP_5005), *phaD* (PP_5006), *phaF* (PP_5007) and *phaI* (PP_5008). In the strain of the invention, the whole *pha* cluster is deleted. The strain comprising the whole *pha* cluster deleted will be also referred to in the present invention as *"P. putida* Δ*pha".*

The "synthetic operon" is a genetic construct, preferably a DNA construct, wherein the genetic material of interest is introduced to be expressed in the cell. The synthetic operon comprises all the elements needed for the expression of the nucleotide sequences of the genes of interest comprised in it. The elements comprised within the genetic construct are in reading frame or "operably linked", i.e. sequentially oriented there between in such a manner as to allow them to function in the intended manner, allowing that the expression of the encoding sequence(s) takes place in compatible conditions with the other elements or sequences present in the construct. In the present invention, the nucleotide sequences of the genes comprised in the synthetic operon are preferably disposed in tandem.

The synthetic operon of the present invention provides for the optimized expression of the genes driving the PHA production. These genes, comprised in the operon, are *phaC1* (PP_5003), *phaF* (PP_5007), *phaG* (PP_1408) and PP_0763.

In a preferred embodiment, the synthetic operon used in the present invention further comprises at least one promoter and at least one terminator. More preferably, the promoter is a constitutive promoter, even more preferably the promoter is SynPro16 (Tiso, T., et al., 2016, Metab. Eng. Commun., 3: 234-244) and the terminator is selected from the list consisting of: λT0, λT1, rnpB-T1 and rpoC-term, or any combination thereof, preferably the terminators used are λT1, rnpB-T1 and rpoC-term.

More preferably, the synthetic operon comprises 4 synthetic modules comprising, each of them, the nucleotide sequence of the gene (*phaC1* gene, *phaF* gene, *phaG* gene or PP_0763 gene) under the synthetic constitutive SynPro16 promoter with an upstream λT0 terminator followed by a standard RBS sequence.

Preferably, the λT0 terminator comprises the SEQ ID NO: 8, the SynPro16 promoter comprises the SEQ ID NO: 9, the standard RBS sequence comprises the SEQ ID NO: 10, the AT1 terminator comprises the SEQ ID NO: 11, the rnpB-T1 terminator comprises the SEQ ID NO: 12 and the rpoC-term terminator comprises the SEQ ID NO: 13. Other alternative promoters that may be comprised in the synthetic operon include, but not limited to, *E. coli trp, recA, lacZ, lacI, tet, gal, trc,* or *tac* gene promoters, the *B. subtilis* α-amylase gene promoter and the *P. putida* promoter Pm.

The synthetic operon can be introduced in a cloning or expression vector to enable its replication and expression in the interior of the cell. In addition to the synthetic operon, the vector comprises other genetic elements operably linked in such a manner that the synthetic operon may be transcribed and translated in the cell. Said genetic elements may be, but not limited to, a promoter, a terminator, a leader sequence, a transcription initiation site, one or more replication origins, a 3' and/or 5' untranslated regulatory region, a potentiator, a polyadenylation signal or any other control sequence. Thus, in another preferred embodiment, the synthetic operon is comprised in a plasmid. More preferably, said plasmid further comprises a vector with ColE1 and/or pVS1 origins of replication (oris). Plasmids with higher copy numbers could be also used such as those including ColE1 and RK2 oris.

The plasmid may be a self-replicating vector, whose replication is independent from the genome of the cell in which it is introduced, or may be a plasmid which becomes integrated in the genome of the target cell once transformed and replicates therewith. Thus, the plasmid may comprise sequences that facilitate its insertion in a specific location within the genome of the host cell.

The synthetic operon may also comprise one or more marker genes whose expression products can be identified, detected and/or quantified due to the fact that they provide an identifiable change in the cell phenotype. Thus, the presence and expression of the marker gene in the synthetic operon used in the invention will make it possible to easily identify the cells that comprise said operon. Said marker gene may be, for example, a gene that confers resistance to metals or medicaments, preferably antibiotics, such as for example genes conferring resistance to kanamycin, neomycin, puromycin, hygromycin, DHFR, GPT, zeocin, histidinol, streptomycin, ampicillin, gentamicin or similar. Inducible or non-inducible reporter genes, such as GFP, EGFP, mCherry, luciferase, IRV3, or any other fluorescent or chemo or bioluminiscent protein, beta-galactosidase, chloramphenicol acetyltransferase (CAT), horseradish peroxidase (HRP) or similar, may also be used. The selection of the marker gene is not relevant provided that it can be simultaneously expressed with the encoding nucleotide sequences for the genes of interest.

In another preferred embodiment, the synthetic operon further comprises at least one antibiotic resistance gene as a marker gene, preferably a kanamycin resistance gene.

The synthetic operon is transformed into the *P. putida Δpha* strain.

Techniques for transforming the *P. putida Δpha* strain with the synthetic operon may be, for example, injection or microinjection, *ex vivo* transformation, electroporation, precipitation with calcium phosphate, DEAE-dextrane followed by polyethylene glycol, sonication, biolistics, retroviral infection, liposome-mediated transfection (lipofection) or via receptor. Preferably, the transformations in the present invention are carried out by electroporation.

The *P. putida* strain comprising the *pha* cluster deleted and the synthetic operon comprising the nucleotide sequences of the *phaC1* gene, the *phaF* gene, the *phaG* gene and the *PP_0763* gene, will be also referred to in the present invention as "MT2" strain.

In another preferred embodiment, the strain of the invention further comprises deleted the following genes: *mmgF* (PP_2334), *scpC* (PP_0154), *sucC* (PP_4186), *sucD* (PP_4185), *aceA* (PP_4116), *glcB* (PP_0356), *icd* (PP_4011), *PP_3190, ocd* (PP_4431), *hutF* (PP_5036) *and PP_3533.* The identification and deletion of these genes in the present invention is the result of applying studies on metabolic models, thus these deletions are model-driven deletions. This strain will be also referred to in the present invention as "MT1" strain.

In another preferred embodiment of the strain of the invention, the synthetic operon further comprises the nucleotide sequences of the *aceF* (PP_0338) (preferably SEQ ID NO: 5), the *aceE* (PP_0339) (preferably SEQ ID NO: 6) and the *lpdG* (PP_4187) (preferably SEQ ID NO: 7) genes. These genes constitute the whole Pyruvate Dehydrogenase Complex (PDH). More preferably, the synthetic operon further comprises 3 synthetic modules comprising, each of them, the nucleotide sequence of the gene (*aceF* gene, *aceE* gene or *lpdG* gene) under the synthetic constitutive SynPro16 promoter with an upstream λT0 terminator followed by a standard RBS sequence.

The strain of the invention that comprises the *pha* cluster deleted and the synthetic operon comprising the nucleotide sequences of the *phaC1* gene, the *phaF* gene, the *phaG* gene, the PP_0763 gene, the *aceF* gene, the *aceE* gene and the *IpdG* gene, will be also referred to herein as "MT7" strain, and it is the most preferred strain of the invention.

The strain of the invention that comprises the *pha* cluster deleted, the synthetic operon comprising the nucleotide sequences of the *phaC1* gene, the *phaF* gene, the *phaG* gene, the PP_0763 gene, the *aceF* gene, the *aceE* gene and the *IpdG* gene, and which further comprises deleted the following genes: *mmgF, scpC, sucC, sucD, aceA, glcB, icd, PP_3190, ocd, hutF and PP_3533*, will be also referred to in the present invention as "MT6" strain.

Other aspect of the invention refers to the use of the strain of the invention for the production of polyhydroxyalkanoate (PHA), preferably for the production of PHA in the absence of nutrient (preferably, nitrogen) limitation.

In a preferred embodiment, the carbon source used by the strain of the invention for the production of PHA is an aromatic compound, sugars, fatty acids, glycerol, a combination of glucose and glycerol, a combination of an aromatic compound and fatty acids, or a combination of sugars and fatty acids.

In a more preferred embodiment, the aromatic compound is selected from the list consisting of: phenyl acetic acid, ferulic acid, *p*-coumaric acid, vanillic acid, 4-hydroxybenzoic acid or any combination thereof. Even more preferably, the aromatic compound is 4-hydroxybenzoic acid (4HBA).

In another preferred embodiment, the sugar is glucose.

In another preferred embodiment, the fatty acid is octanoate.

Another aspect of the invention refers to the use of the MT1, MT2 or MT6 strain of the invention for the production of PHA from glucose or an aromatic compound, preferably 4HBA.

Another aspect of the invention refers to the use of the MT7 strain of the invention for the production of PHA from sugar or a combination of sugar and fatty acids, preferably octanoate.

Another aspect of the invention refers to the use of the MT1, MT6 or MT7 strain of the invention for the production of PHA from a combination of an aromatic compound, preferably 4HBA, and fatty acids, preferably octanoate.

Another aspect of the invention refers to a method, hereinafter "the method of the invention", for the production of PHA, preferably for the production of PHA in the absence of nutrient (preferably, nitrogen) limitation, comprising the following steps:
a. incubating the strain of the invention with a carbon source, and
b. recovering the PHA produced after the incubation of step (a).

Preferably, step (a) of the method of the invention is carried out at a temperature in a range between 25 and 37°C, more preferably 30°C, during 20 to 72 h, more preferably 24h, under vigorous shaking at, preferably, 200 rpm, in the presence of the culture medium, preferably the M63 medium (13.6 g of KH₂PO₄/L, 2 g (NH₄)₂SO₄/L, 0.5 mg FeSO₄ •7H₂O/L, adjusted to pH 7.0 with KOH), supplemented with the carbon source and with 1 mM MgSO₄ and a solution of trace elements. Preferably, such solution of trace elements comprises a composition 1000 X dissolved in 1N HCl: 2.78 g/L of FeSO₄•O7H₂O, 1.98 g/L of MnCl₂•4H₂O, 2.81 g/L of CoSO₄•O7H₂O, 1.47 g/L CaCl₂•2H₂O, 0.17 g/L of CuCl₂•2H₂O, 0.29 g/L of ZnSO₄•7H₂O. Other examples of culture media useful for the incubation of step (a) are M9 minimal medium and Luria Broth.

Preferably, when the carbon source used in the method of the invention comprises an aromatic compound, 0.034 mM of EDTA is added to the culture medium.

In a preferred embodiment of the method of the invention, the carbon source is an aromatic compound, sugars, fatty acids, glycerol, a combination of glucose and glycerol, a combination of an aromatic compound and fatty acids, or a combination of sugars and fatty acids.

In a more preferred embodiment, the aromatic compound is selected from the list consisting of: phenyl acetic acid, ferulic acid, *p*-coumaric acid, vanillic acid, 4-hydroxybenzoic acid or any combination thereof. Even more preferably, the aromatic compound is 4-hydroxybenzoic acid (4HBA).

In another preferred embodiment, the sugar is glucose.

In another preferred embodiment, the fatty acid is octanoate.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

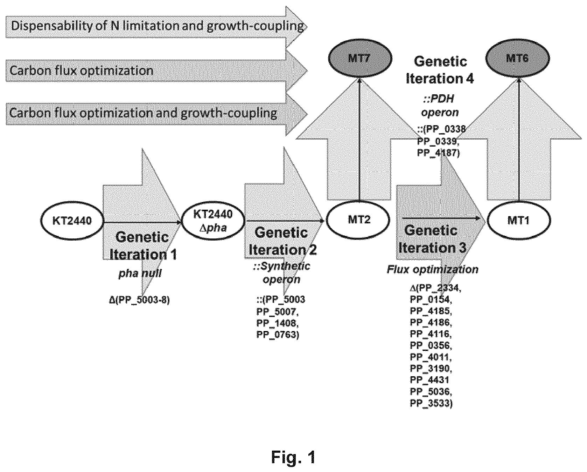

**Fig. 1****.** Workflow of the construction of the strains of the invention.

### EXAMPLES

### Example 1. Strains and culture media. Methods.

The strains and plasmid used in this invention are described in Table 1 below.

**Table 1**

| **Strains** | **Phenotype/ Relevant characteristics** |
|---|---|
| ***P. putida*** | |
| KT2440 | Rf^{r}, TOL plasmid-cured, spontaneous restriction deficient derivative of *P. putida* mt-2 (Bagdasarian, M., et al., 1981, Gene 16: 237-247) |
| KT2440 Δ*pha* | KT2440 derivative strain, *pha* cluster deletion mutant (https://www.ncbi.nlm.nih.gov/sra/?term=SRX3133083) |
| MM19 | KT2440 derivative strain, Δ(*mmgF scpC sucC sucD aceA glcB icd PP_3190 ocd hutF PP_3533 pha* cluster) |
| **MT1** | **Km^{r}, MM19 strain with pMM55** |
| **MT2** | **Km^{r}, KT2440 Δ*pha* strain with pMM55** |
| **MT6** | **Km^{r}, MM19 strain with pMM62** |
| **MT7** | **Km^{r}, KT2440 Δ*pha* strain with pMM62** |
| ***E. coli*** | |
| DH5a | Cloning host; F⁻ λ⁻ *endA1 glnX44*(AS) *thiE1 recA1 relA1* spoT1 *gyrA96*(Nal^{R}) *rfbC1 deoR nupG purB20* Φ80(*lac*ZΔM15) Δ(*lacZYA-argF*)U169, hsdR17(*rK⁻mK*⁺⁾, (Grant, S.G., et al., 1990, Proc. Natl. Acad. Sci., 87(12): 4645-9). |
| DH10B | Cloning host; F-, *mcrA* Δ(*mrr hsdRMS-mcrBC*) ϕ80d*lac*ΔM15 Δ*lacX74 deoR recA1 araD139* Δ(*ara*-*leu*)7697 Invitrogen, Thermo Fisher Scientific, USA |
| HB101 | Helper strain; F⁻*λ⁻ hsdS20(r_{B}⁻ m_{B}⁻) recA13 leuB6*(Am) *araC14*Δ(*gpt-proA*)62 *lacY1 galK2*(Oc) *xyl-5 mtl-1 rpsL20*(Sm^{R}) *glnX44*(AS) (Boyer, H.W., et al., 1969, J. Mol. Biol., 41(3): 459-72). |
| | |

| **Plasmids** | **Relevant characteristics** |
|---|---|
| pRK600 | Cm^{r}, ColE1 *oriV* RK2 Mob⁺ Tra⁺ donor of transfer functions (Kessler, B., et al., 1992, Mol. Gen. Genet., 233(1-2): 293-301). |
| pK18*mobsacB* | Km^{r}, pMB1, *oriV*, Mob⁺, *lacZa, sacB*; vector for allelic exchange homologous recombination mutagenesis (Schäfer, A., et al., 1994, Gene, 145(1): 69-73). |
| **pMM55** | **Km^{r}, ori ColE1/pVS1, golden gate cloning vector level 2; *phaC1*; *phaF*; dummie 3; *phaG*; PP_0763** |
| **pMM62** | **Km^{r}, ori pMB1/ColE1, golden gate cloning vector level 2; *phaC1*; *phaF*; dummie 3; *phaG*; PP_0763; *aceF*; *aceE*; *lpdG*** |

The workflow of the construction of the strains of the invention is shown in Fig. 1.

The culture media used in this invention are those described below.

*E. coli* and *P. putida* strains were grown routinely for DNA manipulations and for pre-cultures in lysogeny broth (LB) medium at 37°C and 30°C, respectively. The appropriate selection antibiotics, gentamicin (10 µg/ml), chloramphenicol (34 µg/ml), ampicillin (100 µg/ml), kanamycin (50 µg/ml), streptomycin (75 µg/ml), IPTG (0.5-1 mM) and Xgal (40 µg/ml) were added when needed.

Standard growth experiments of *P. putida* in defined medium were performed in M63 minimal medium (13.6 g of KH₂PO₄/L, 2 g (NH₄)₂SO₄/L, 0.5 mg FeSO₄•7H₂O/L, adjusted to pH 7.0 with KOH), plus the carbon source needed in each case. The medium was supplemented with 1 mM MgSO₄ and a solution of trace elements (Goodies) (composition 1000 X dissolved in 1N HCl: 2.78 g/L of FeSO₄•7H₂O, 1.98 g/L of MnCl₂•4H₂O, 2.81 g/L of CoSO₄•7H₂O, 1.47 g/L CaCl₂•2H₂O, 0.17 g/L of CuCl₂•2H₂O, 0.29 g/L of ZnSO₄•7H₂O). For growth on aromatic compounds, 0.034 mM of EDTA was added to the medium.

### 1.1. Synthetic Biology DNA Techniques

Standard molecular biology techniques were performed. Plasmid DNA Isolation was made using the High Pure Plasmid Isolation Kit (Roche, Mannheim, Germany) following the manufacturer's protocol. Genomic extractions of *P. putida* KT2440 were performed with the illustraTM bacteria genomicPrep Mini Spin Kit (GE Healthcare, Buckinghamshire, UK) or PureLink® Genomic DNA kit (Invitrogen). DNA agarose gel bands, PCR products and digestion products were purified with illustraTM GFX PCR DNA and Gel Band Purification Kit (GE Healthcare, Buckinghamshire, UK) or QIAquick Gel Extraction Kit (Quiagen) and QIAquick PCR Purification Kit (Quiagen). DNA concentrations were measured with Nano Drop® 2000 Spectrophotometer (Thermo Scientifc, Massachusetts, USA). The enzymes Phusion DNA polymerase, T4 DNA ligase, EcoRI-HF, BamHI-HF, XbaI, HindIII-HF, NotI-HF, NcoI-HF, DraIII-HF, PacI, SpeI and CutSmart buffer were acquired from New England Biolabs (NEB, Ipswich, Massachusetts). For cloning confirmation, the DNA Taq polymerase (Biotools, Madrid, Spain) was used. The GoldenGate-MoClo enzymes, BpiI and BsaI, and Buffer G were acquired from Thermo Scientific (Massachusetts, USA). The GoldenGate-MoClo Toolkit (Kit 1000000044) was purchased from Addgene (Massachusetts, USA).

### 1.1.1. Preparation and transformation of competent strains

Preparation of chemically competent *E*. *coli* cells was carried out using the RbCI method and their transformation was followed.

*P. putida* strains were transformed following the Choi *et al.* protocol with some modifications (Choi, K.H., et al., 2006, J. Microbiol. Methods, 64(3): 391-7). *P. putida* KT2440 strains were grown overnight in 20 ml of LB at 30°C. These cultures were pelleted at 3000 x*g* for 20 minutes at 4°C, washed five times with 300 mM sucrose and suspended in 500 µl of 300 mM sucrose. 100 µl of cell suspension was mixed with 100 ng of desired plasmid and transferred to a 2 mm gap electroporation cuvette. After a pulse of 25 µF, 2.5 kV and 200 Ω (Gene Pulser/Pulse Controller (Bio-Rad)), 900 µl of fresh room temperature LB was added and transferred to a 100 x 16 mm round-bottom polypropylene tube and incubated for 1 hour at 30°C and 200 rpm. Different dilutions were plated on LB 1.5% agar medium with corresponding antibiotic selection and grown at 30°C for 18 hours.

### 1.1.2. Plasmid transfer by conjugation

To perform a conjugation transfer, it is needed to bring together the donor cell that bears the plasmid of interest, the recipient strain which is the target bacterium and a helper bacterial strain to assist and catalyze the mating process. The mating helper is an *E. coli* strain that provides the conjugation machinery which is normally derived from a RK2 or RP1 plasmid that involves the mobilization (*mob*) and transfer (*tra*) functions supplied *in trans.* There are two basic types of helper strains, which express the above-mentioned functions either on a plasmid (such as HB101 carrying pRK600) or genome integrated (such as S17λ*pir*). In this invention, the former type of helper strain was used, and three bacterial strains in the mating process need to be included, offering the possibility of changing the donor *E. coli* strain. The donor strain should contain the *pir* gene as *λpir* lysogen such as CC118λ*pir* or DH5aλ*pir* in order to favor counter selection of transconjugants as needed.

Triparental mating was performed using *E. coli* DH10B with pMM series plasmids as donor strain, *E. coli* HB101 pRK600 as helper strain and *P. putida* KT2440 as recipient strain. The LB pre-inoculum strains were washed twice using 0.85% saline solution and then 100 µl of each strain were mixed in an Eppendorf. As a negative control, the mixed strains without the recipient one was used. Then, on nonselective LB agar plate, we placed filters of 0.22 µm pores (Merck) and 100 µl of the mixture of the strains were placed on top of the filters. The plate was incubated for 10 hours in 30°C. Placing the conjugation mixture on a solid support facilitates *oriT*-mediated conjugation by immobilizaing cells in close proximity to one another. The filter was used to facilitate the recovery of cells, and nonselective medium was required to avoid killing non-transformed recipient cells. The counterselection against *E. coli* was achieved by using *Pseudomonas*-selective rich media such as cetrimide-containing agar or minimal M63 medium supplemented with 0.2% citrate or 5 mM benzoate (*E*. *coli* donor cells cannot use citrate or benzoate as sole carbon and energy source). In all the cases, the corresponding antibiotic was added in order to inactivate any non-transformed *P. putida* cells.

### 1.1.3. Construction of P. putida deletion mutants

Two deletion strategies, the *pK18mobsacB* and pEMG strategy, were used.

The gene of interest was inactivated by allelic exchange homologous recombination using the mobilizable plasmid *pK18mobsacB.* The PCR primer pairs were designed in order to amplify approximately 500-800 bps regions upstream (Z1) and downstream (Z3) of the gene that will be deleted to serve as recombination arms of homology. It was added proper restriction enzyme sites upstream of Z1 and downstream of Z3 in order to clone the fragment Z1Z3 into *pK18mobsacB.* The two purified fragments Z1, Z3 were used in order to obtain a Z1Z3 fusion fragment (overlapping PCR). The obtained Z1Z3 fragment and the plasmid *pK18mobsacB* were digested with the appropriate restriction enzymes and further gel purified. Finally, the purified fragment and plasmid were ligated using T4 DNA ligase (New England Biolabs) overnight at 16°C. These deleted genes were cloned into the corresponding unique sites of pK18*mob*sacB plasmid and transformed into chemically competent *E*. *coli* DH10B. The transformants were plated on LB plates with kanamycin (50 µg/ml), 0.5 mM IPTG and 40 µg/ml Xgal for selection and blue/white screening. Colony PCR was used to verify selected white colonies using the F24, R24 primer pair. From selected candidates we performed plasmid isolation and the correct sequence of the cloned inserts was confirmed by DNA sequencing.

The resultant plasmid was used to delete the target gene to the host chromosome via homologous recombination. Triparental mating was performed using *E. coli* DH10B with pMM series of plasmids as donor strain, *E. coli* HB101 pRK600 as helper strain and *P*. *putida* KT2440 as recipient strain (see Table 1 for more details about the strains and plasmids used in this invention). For the first recombination selection process the cells were plated on cetrimide agar containing 50 µg/ml kanamycin, that permitted only the selection of *P. putida* strains. The resulted recombinant strains were confirmed by PCR and the selected colonies were grown in LB during 6 hours and then plated on M63 10 mM citrate selective plates supplemented with 5% sucrose. The *sacB* counterselection marker from *Bacillus subtilis* confers sucrose sensitivity to Gram negative bacteria. Almost all sucrose-resistant colonies will have been cured from *pK18mobsacB* plasmid. Transconjugants sucrose resistant and kanamycin sensible were isolated and the second crossover event was confirmed by PCR using external primers of the arms of homology region (for example Z1-For, Z3-Rev) and DNA sequencing.

For the *pha* cluster deletion, the pEMG knockout system was used, with some modifications. The pEMG system is based on similar principles of markerless gene replacements by a double-strand break in the chromosome than the *pK18mobsacB.* As far as it concerns the design of the homology regions (called as TS1 and TS2 regions, upstream and downstream of to be deleted gene (s), respectively), the delivery vector (pEMG) and the first recombination event transconjugants are the same as in the case of *pK18mobsacB* strategy.

The key event in the procedure is the generation of a single break in target genome through conditional expression of the I-Sce-I endonuclease. The I-Sce-I sites are entered in the chromosome of the bacterium upon co-integration of the recombinogenic vector. Under these conditions, surviving cells must have recombined the sequences that flank the I-Sce-I site to restore chromosomal integrity. The constructing vector pEMG incorporates two I-Sce-I sites flanking a *lacZa* poly-linker. This vector functions in concert with the I-Sce-I producing pSW(I-SceI) plasmid that allows the accumulative edition of the genome of *P. putida.*

Thus, the *P. putida* transconjugants were transformed of the first recombination event with pSW-I (Amp^{r}, I-Sce-I expression vector) via electroporation. The selection plates were LB + 500 µg/ml Amp and 15 mM 3-methylbenzoate (3MB) and incubated on 30°C for 16 h. We should note here that KT2440 is naturally resistant to ampicillin but such concentration allows plasmid selection. Additionally, the I-Sce-I endonuclease expression is dependent on a 3MB inducible promoter system. Single colonies were picked on LB + 500 µg/ml Amp and LB + Km to screen for kanamycin sensitive clones. The following step was to screen kanamycin sensitive clones for the desired knockout (since the wild type situation can be restored) performing a colony PCR using external primers to the homology region (FdPHA and RdPHA) and by DNA sequencing of the PCR product. Under non-selective cultivation *Pseudomonas* loses the pSW-I plasmid quite fast. Finally, several single colonies were checked for 500 µg/ml Amp sensitivity to verify its loss.

### 1.1.4. GoldenGate-MoClo protocol

Golden Gate cloning method is based on the use of type IIS restriction enzymes combined with restriction-ligation. Extremely high cloning efficiency is obtained using a simple one-pot incubation of multiple undigested entry constructs and a destination vector in the presence of restriction enzyme and ligase. MoClo (modular cloning system) allows any multigene construct of choice to be made by using a defined set of pre-made vectors and a defined assembly strategy. Basic genetic elements like promoters, coding sequences and terminators are cloned at level 0 modules. Each module is flanked by two Bsal restriction sites specific for each modules type, with cleavage sites designed to allow sets of compatible modules to be assembled in one step in a level 1 destination vector (using Bsal enzyme). The resulting level 1 constructs contain transcriptional units, which are then assembled six at a time in level 2 constructs using a second type IIS enzyme, Bpil. The cloning process can then be repeated to add more transcription units to the resulting construct by alternating the type IIS enzyme (s) for cloning.

GoldenGate-MoClo plasmids were constructed. For Level 0 plasmid construction, every part was PCR amplified with oligonucleotides designed with the Benchling platform (www.benchling.com) with the following characteristics: a tail containing the Bpil recognition site followed by the corresponding 4-nt fusion site, 21 bp of minimal length for target complementarity, 50°C of minimal Tm for that region and a maximal Tm difference of ± 1.5°C between both oligonucleotides. If a part contained a Bpil/Bsal recognition site, it would be eliminated introducing by PCR amplification silent samesense point mutations in the restriction site sequences. PCR products were purified using gel purification kit following manufacturer instructions. Digestion-ligation reaction was set up by pipetting in one tube 100 ng of destination vector and the corresponding DNA amount of PCR product to maintain 2:1 insert-vector molar ratio, 10 U of Bpil, 400 U of T4 DNA ligase and 1 mM ATP in commercial Buffer G in a final reaction volume of 20 µl. The reaction was incubated in a thermocycler for four cycles of restriction-ligation at 37°C for 10 minutes and 16°C for 10 minutes and then a heat inactivation at 65°C for 20 minutes. The final step is of great importance, thus, omitting this inactivation would lead to re-ligation of some of the insert and plasmid backbone fragments when the reaction vessel is taken out of the thermal cycler and would lead to a higher proportion of colonies containing incorrect constructs 100 µl of chemically competent *E. coli* DH5α were transformed with 5 µl of reaction mix. Transformed cells were grown overnight at 37°C in LB 1.5% agar supplemented with 75 µg/ml streptomycin, 0.5 mM IPTG and 40 µl/ml X-gal. Blue-white selection was carried out and four white colonies were transferred to 4 mL of liquid LB medium with 75 µg/ml streptomycin for plasmid purification. The extracted plasmids were digested for an hour at 37°C with Bsal to check the presence of the correct insert in an agarose 0.7% gel. Two plasmids with the correct insert size were sequenced with RK81-RK82 to verify sequence integrity.

The position and orientation of each gene in a final construct determine which level 1 destination vector has to be chosen for assembly of a transcription unit. A total of 14 vectors are available (pL1F1-7, pL1R1-7) for cloning of genes either forward or reverse orientation at each of seven possible positions. These level 1 vectors differ only by the sequence of the fusion sites. The two external fusion sites (Bpil cleavage sites) of each vector are designed to be compatible with the fusion sites of the vectors from the position before and after. The downstream fusion site of the vector at position 7 is compatible to the upstream fusion site of vector at position 1 (TGCC). Thus, multigene constructs with more than seven genes can be assembled without the need for additional cloning vectors. In practice, a gene that is planned to be cloned at position 8 in a multigene construct is simply cloned in a position 1 destination vector etc. For Level 1 plasmid construction of transcription units, the reaction mix was composed by 100 ng of destination vector (pL1F-1 to pL1F-7 depending on the CDS position), a promoter plasmid part, a CDS plasmid part and a terminator plasmid part in a 2:1 donor vector-destination vector molar ratio. 10 U of Bsal, 400 U of T4 DNA ligase and 1 mM ATP in Buffer G in a final reaction volume of 20 µl. The reaction was incubated in a thermocycler for four cycles at 40°C for 10 minutes and 16°C for 10 minutes, then at 50°C for 10 minutes and heat inactivation at 80°C for 20 minutes. 100 µl of chemically competent *E*. *coli* DH10B were transformed with 5 µl of the reaction. Transformed cells were grown overnight at 37°C in LB 1.5% agar supplemented with 100 µg/ml ampicillin, 0.5 mM IPTG and 40 µl/ml X-gal. Blue-white selection was carried out and four white colonies were transferred to 4 mL of liquid LB medium with 100 µg/ml ampicillin to do a plasmid purification. The extracted plasmids were digested for an hour at 37°C with Bpil to check the presence of the correct insert in a 0.7% agarose gel. Two plasmids with the correct insert size were sequenced with RK155-RK156 to verify sequence integrity.

Level 2 vectors contain two inverted Bpil recognition sites for insertion of level 1 modules. The upstream fusion site (TGCC) is compatible to a gene cloned in a level 1 vector, whereas the downstream fusion site consists of a universal sequence (GGGA). This design allows cloning of two to six genes in the same vector. Using more genes in this step would lead to incorrect clones because the same fusion sites would be present in different modules. The last gene is fused to the vector by using a compatible linker. There are two types of linkers, the "closed" construct, where no further genes can be added (pELE-1-7) and the "opened" construct for cloning of further genes (pELB1-7 and pELP1-7). Level 2 reactions were carried out with 100 ng of destination vector (pL2), the corresponding end-linker vector (depending on the number of transcriptions units and if the construct was "opened" or "closed"), the vector/s with each transcription unit in a 2:1 donor vector-destination vector molar ratio. 10 U of Bpil, 400 U of T4 DNA ligase and 1 mM ATP in Buffer G in a final reaction volume of 20 µl. The reaction was incubated in a thermocycler for four cycles of 37°C for 10 minutes followed by at 16°C for 10 minutes, then heat inactivation at 65°C for 20 minutes. 100 µl of chemically competent *E*. *coli* DH10B were transformed with 5 µl of the reaction. Transformed cells were grown overnight at 37°C in LB 1.5% agar supplemented with 50 µg/ml kanamycin. Red-white selection was carried out and four white colonies were transferred to 4 mL of liquid LB medium with 50 µg/ml kanamycin to do plasmid purification. The extracted plasmids were digested for an hour at 37°C with DraIII-HF and other control enzymes in order to check the presence of the correct inserts in an agarose 0.7% gel. During each step of the part construction, apart from the control digestion process, sequencing of all the included parts was realized.

### 1.2. Systems Biology Methods

### 1.2.1 Constraint-based flux analysis

The *i*JN1411 model was exported from SimPHeny as an SBML file and analysed with the COBRA Toolbox v2.0 within the MATLAB environment (The MathWorks Inc.). Tomlab CPLEX and the GNU Linear Programming kit were used for solving the linear programming problems. The constraint-based model consists of a 2087 x 2826 matrix containing all the stoichiometric coefficients in the model of 2087 metabolites and 2826 reactions (S).

Flux balance analysis (known as FBA) is a widely used approach for studying biochemical networks, in particular GENREs (genome-scale metabolic networks reconstructions). FBA calculates the flow of metabolites through the given metabolic network. Thus, FBA makes possible to analyze the phenotypes and capabilities of organisms with different environmental or genetic perturbations, to predict the growth rate of an organism or the rate of production of a metabolite of interest.

FBA is based on solving a linear optimization problem by maximizing or minimizing a given objective function Z subject to a set of constraints. The constraints S*v=0 correspond to a situation of steady-state mass conservation where the change in concentration of the metabolites as a function of time is zero. The vector v represents the individual flux values for each reaction. These fluxes are further constrained by defining lower and upper limits for flux values. For reversible reactions an upper and lower bound of-1000 mmol.gDW⁻¹.h⁻¹ and 1000 mmol.gDW⁻¹.h⁻¹ were used respectively. A lower bound of 0 mmol.gDW⁻¹.h⁻¹ was used in case of irreversible reactions. For simulating condition-specific growth conditions, lower bounds of the corresponding exchange reactions were modified accordingly.

In FBA the metabolic reactions equations are solved using computational linear programming algorithms such as the COBRA (constraint-based reconstruction and analysis) Toolbox (is a freely available MATLAB toolbox). COBRA emphasizes describing the constrains that a system must satisfy rather than computing an explicit solution, by leading to the definition of a "solution space", which contains the set of feasible solutions that satisfy all imposed constraints. Some of the limitations of FBA are the fact that cannot predict the metabolite concentrations since it does not use kinetic parameters and it is only suitable for determining fluxes at steady state.

### 1.2.2 Growth-coupled overproducing strains

For growth-coupled overproducing strain designs, the Genetic Design through Local Search (GDLS) algorithm was used. GDLS is a computational design tool for metabolic engineering that uses an efficient, low-complexity local search approach to identify favourable genetic designs using flux-balance metabolic models. GDLS was chosen over other strain design methods due to the complexity of our objective, e.g., to find growth-coupled designs of PHA overproducing strains from PHA-unrelated substrates, which could anticipate a large number of gene knockouts needed, and would require a large computational runtime due to the size of our model. GDLS is a heuristic algorithm method capable of handling large metabolic models while allowing a much larger number of genetic manipulations in the final design than other methods. In addition, GDLS runtime scales linearly in contrast to other globally-optimal search methods that scale exponentially. Moreover, GDLS employs a local search approach with multiple search paths to find a set of locally optimal strategies that allow for the analytical evaluation of the different paths. Similar to other strain design methods, GDLS implements reductions that simplify FBA models. Finally, GDLS was demonstrated to have as good performance as other globally optimal search methods such as OptFlux and Optknock with an order of magnitude of improvement in computational time for solutions that yield equal or comparable value. The function GDLS implemented in COBRA 2.0 was used.

In order to achieve an optimal biomass-coupled product yield (BCPY), GDLS runs were computed using minimum growth rate values up to 60% of that predicted for the wild-type model. Due to computing time limitations, the number of simultaneous knockouts (neighborhood parameters) tested was 2 to 4, and 3 was the value that showed a significant increase in PHA production in all designs described in the results. The number of paths used was tested from 1 to 10 depending of the computing complexity for each neighborhood value. Generally, a single path was selected by default for each case described in the results. The maximum number of knockouts and iterations were set to 50 and 70, respectively, by default. Those paths with the greatest BCPY value in the final design for each case were selected.

### 1.3. Analytic Methods

### Total biomass calculation

40 ml of culture medium were centrifuged, in previously tared 50 ml Falcon tubes, for 45 min at 3000 *xg* at 4°C. Cell pellets were rapidly frozen at -80°C and were freeze-dried for 24 h in a lyophilized. Finally, the tubes were weighted and the cell densities were expressed in grams of cell dry weight (CDW) per liter. By residual biomass, it is referred to the total biomass free of PHA.

### Methanolysis process and GC-MS analysis for PHA determination

For composition and cellular PHA content, standard GC-MS techniques of the methanolysed polyester with some modifications were used. 2-5 mg of lyophilized samples (culture pellets) were resuspended in 2 ml of methanol containing 15% sulfuric acid (Sigma-Aldrich, Merck, Germany) and 2 ml of chloroform containing 0.5 mg/ml 3-methylbenzoic acid (internal standard; Sigma-Aldrich, Merck, Germany) and then incubated using screw-capped tube at 100°C for 5-6 h. After cooling, 1 ml of distilled water was added to the mixture, to extract most cell debris and the sulfuric acid left. A two-phase extraction process was performed by removing completely the water phase to prevent fouling of the GC column. Finally, a small amount of Na₂SO₄ powder was added to dry the chloroform phase and to remove any water remains. The absence of sulfuric acid residues to the samples was verified by checking that the pH was 5. Then, the organic phase containing the resulting methyl esters of monomers was analyzed by GC-MS.

An Agilent (Waldbronn, Germany) series 7890A coupled with a 5975C MS detector (El, 70 eV) and a split-splitless injector was used for analysis. An aliquot (1 µl) of organic phase was injected into the gas chromatograph at a split ratio of 1:50. During this work the DB-5HTDB-5HT column (400°C: 30 m x 0.25 mm x 0.1 µm film thickness) was used. Helium was used as carrier gas at a flow rate of 0.9 ml min⁻¹. The injector and transfer line temperature were set at 275°C and 300°C respectively. The oven temperature program was initial temperature 80°C for 2 min, then from 80°C up to 175°C at a rate of 5°C min⁻¹, for efficient separation of peaks. El mass spectra were recorded in full scan mode (*m*/*z* 40-550). With this program, monomers from C4 to C14 were detected. The retention time for each monomer is 1.9 min (C4), 2.4 min (C5), 3.5 min (C6), 4.8 min (C7:1), 7.2 min (C8), 9.3 min (C9:1), 12.0 min (C10), 14.1 min (C11:1), 16.2 min (C12:1), 16.6 min (C12), 20.9 min (C14) and 6.1 (3MB, internal standard).

### Optical Microscopy

Cultures were routinely visualized with a 100 X phase-contrast objective (Nikon microscope) and images were taken with a connected camera Leica DFC345 FX.

### Transmission Electron Microscopy

*P. putida* cells previously grown under the conditions of interest for 24h were harvested and washed twice in 1 X PBS. Afterwards, cells were suspended in 2.5% (w/v) OsO₄ for 1 h, gradually dehydrated in ethanol (30, 50, 70, 90, and 100% (v/v); 30 min each) and propylene oxide (1 h), embedded in Epon 812 resin. Ultrathin sections (thickness 70 nm) were cut with a microtome using a Diatome diamond knife. The sections were picked up with 400 mesh cupper grids coated with a layer of carbon and subsequently observed in a Jeol-1230 electron microscope (JeolLtd, Akishima, Japan). The analysis of the biological samples was performed by CIB services.

### Example 2. Recombinant strains construction

The workflow followed for the construction of PHA overproducer strains is as detailed in Figure 1. Four sequential genetic iterations were required to optimize the PHA production in *P. putida.*

### 2.1. Genetic iteration 1

The genetic iteration 1 included the deletion of the genes involved in PHA metabolism, these include *phaC1* (PP_5003), *phaZ*(PP_5004), *phaC2* (PP_5005), *phaD* (PP_5006), *phaF* (PP_5007) and *phaI* (PP_5008). The knocking out of these genes was performed using the pEMG deletion system.

The *pha* metabolism is subjected to a complex regulation including global and specific regulator. In order to avoid this complex regulation, the whole *pha* cluster was deleted using pEMG deletion strategy, resulting in *P. putida Δpha* strain.

### 2.2. Genetic iteration 2, generation of MT2 strain

The genetic iteration 2 involves the construction of a synthetic operon with expression optimized for the genes driving the production of PHA including *phaC1* (PP_5003, SEQ ID NO: 1), *phaF* (PP_5007, SEQ ID NO: 2), *phaG* (PP_1408, SEQ ID NO: 3), and PP_0763 (SEQ ID NO: 4). The synthetic operon was constructed by using Golden Gate assembly (Weber, E., et al., 2011, PLoS One, 6(2): e16765).

In order to allow the PHA production, while avoiding both specific and global events of gene expression regulation, it was decided to construct a synthetic PHA operon including the overexpression of the *phaG* gene and the medium chain-fatty acid CoA ligase (PP_0763). Furthermore, the expression levels of some of the essential *pha* genes needed for the PHA production were included in the synthetic operon, including the key genes involved in the PHA production, such as *phaC1* synthase and *phaF* phasin.

In all the cases, the SynPro16 promoterwas used with an upstream λT0 terminator which was introduced into the Golden Gate-MoClo pL0-PU plasmid. Genes encoding PhaC1, PhaF, PhaG and PP_0763 enzymes from *P. putida* KT2440 were cloned intro the pL0-SC plasmid from the Golden Gate-MoClo kit. The terminator λT1, rnpB-T1 and rpoC-term were cloned into the pL0-T plasmid from the Golden Gate-MoClo kit.

Four Golden Gate-MoClo Level 1 reactions were carried out to create transcription unit constructs for the *phaC1*, *phaF, phaG* and PP_0763. In all the transcription units built the SynPro16 promoter and the corresponding terminators were used. The MoClo system Level 1 plasmids used were pLF-1 (for position 1) to pLF-5 (for position 5), respectively. These positions will allow the correct Level 2 assembly. Correct Level 1 assemblies/plasmids were then mixed with the Level 2 plasmid and the appropriate Level 2 end-linker. The Level 2 Product vector (pMM55) contained an end-linker with the LacZ that maintained the construct opened, allowing the addition of extra transcription units if needed. The pMM55 plasmid was then transformed into KT2440 Δ*pha* strain by electroporation, generating the MT2 strain.

### 2.3. Genetic iteration 3, generation of MT1 strain

Genetic Design through Local Search (GDLS) algorithm and the genome-scale model of *P. putida i*JN1411 were used to identify potential gene deletions resulting in a growth-coupled PHA overproduction strain. Polyethylene terephthalate (PET) was used as carbon source in these *in silico* simulations. The resulting *in silico* growth-coupled designs were subsequently manually curated.

In order to perform the growth-coupling analysis, the most comprehensive metabolic model of *P. putida* available so far, *i*JN1411 was used. *i*JN1411 (https://n9.cl/u7o4) was updated with the metabolic pathways involved in the metabolism of PET via terephthalate (TPHTA) and ethylene glycol (EG). This update resulted in the addition of 32 new reactions and 25 new metabolites (https://n9.cl/zst4). The resulting model, named *i*MM1412, was used for the subsequent analysis.

As a first step, a simplified version of *i*MM1412 was constructed to reduce the computational complexity of the process. The reduced model was constructed by applying two consecutive simplification steps e.g., model simplification and model reduction. Four additional reduction steps were performed and included the removal of: i) reactions encoded by essential and nearly-essential genes, ii) all reactions without associated genes in the model, iii) peripheral and transport reactions, and iv) the sets of flux-coupled reactions were aggregated as a single reaction.

These approaches allowed to significantly reduce the number of reactions suitable to be deleted. This simplified model version included a less complex biomass equation and reduced PHA and alginate metabolism, e.g., only the final production of medium-chain length aliphatic PHA containing 8 carbon atoms (C8) and a specific single alginate (alginate with 3 units of acetylated D-mannuronate and 2 units of L-guluronate) were considered. This simplification together with the reduced model constructed for each carbon source, reduced the complexity of the GDLS analysis. A reduced list of suitable reactions for knockout was finally generated following the above-mentioned steps for each carbon source.

### Model-driven design of PHA overproducing strains using PET

It was first addressed the identification of growth-coupled *in silico* overproducing PHA designs using PET as sole carbon and energy source. PET was used because protocatechuate is an intermediate of its degradation. Therefore, the GDLS algorithm was applied using an uptake rate of PET of 3.78 mmol·gDW⁻¹·h⁻¹ and the default parameters for GDLS. As corresponds with a local search algorithm, in each positive iteration, GDLS keep those deleted reactions and initiate a new iteration, this way an individual path with increasing number of knockouts is provided. Several GDLS paths were identified and that more promising in terms of i) PHA yield, ii) BCPY (Biomass-coupled product yield), iii) number of knockouts and iv) physiological feasibility was selected for further analysis.

As the number of gene knockouts is increased, higher is the production of PHA. It was achieved more than 50% of biomass as PHA in the PET4 design. In order to reach this high PHA production up to 21 reaction knockouts were required.

### Synthetic Biology implementation of in silico PET4 PHA overproducer design

The complete PET4 design was based on the deletion of 21 reactions, therefore making challenge its *in vivo* implementation due to the large number of genetic modifications required. Thus, as a first step, a manual curation of PET4 design was performed in order to identify the minimal number of knockouts still resulting in an efficient growth-coupled design. By this effort, the importance of each reaction towards growth-coupled PHA production was evaluated. Some reactions including RHACOAE80, PHADPC80 among others, showed a dispensable behaviour for PHA production. Furthermore, the UPPN and ALATA_L reactions resulted in slight decrease in PHA content and the PDH reaction showed a moderate decrease in the PHA production. Finally, 9 reactions were identified as essential for growth-coupled PHA production.

Aimed by the essentiality of the reactions for PHA production, it was feasible the identification of three possible growth-coupled designs accounting for the increasing number of knockouts. The minimal design, despite the significant lower number of knockouts required, keeps the ability to overproduce a significant amount of PHA and not so different PHA content to the medium and complete designs.

The minimal's PET4 design was used as a starting point. These individual reactions were blocked sequentially by deleting the corresponding coding gene(s). We relied on a system for individual gene deletions (*pK18mobsacB*) and performed many successive rounds in order to accumulate all the necessary deletions in a single strain. A logical order to delete the genes was planned based on the predicted importance of the pathways to which the reactions belong.

Therefore, the first three reactions deleted were those responsible of blocking in succinate metabolism, 2-methylisocitrate lyase, propionyl-CoA:succinate-CoA transferase and succinyl-CoA synthetase subunit alpha/beta. The next reactions knocked out were responsible of glyoxylate cycle, isocitrate lyase and malate synthase. Glyoxylate cycle is a variation on the TCA cycle in charge of the biosynthesis of 4-carbon metabolites from acetyl-CoA. The subsequent deletion was the isocitrate dehydrogenase, that when taken together with the five previous deletions, it were expected to eliminate a substantial portion of the carbon flux through the TCA cycle. The last deletions were required in order to avoid alternative metabolism of acetyl-CoA and acetate secretion via amino acid metabolism.

The final strain including all these knockouts was called MT1.

### 2.4. Genetic iteration 4

The genetic iteration 4 accounted the construction of a synthetic operon including the overexpression of the whole Pyruvate Dehydrogenase Complex (PDH) e. i., *aceF* (PP_0338, SEQ ID NO: 5), *aceE* (PP_0339, SEQ ID NO: 6) and *IpdG* (PP_4187, SEQ ID NO: 7). The synthetic operon was constructed by using Golden Gate assembly. As a result, strains MT6 and MT7 were constructed.

Using the *in silico* metabolic model *i*JN1411 (wild type) and the *in silico* strains it was analysed the effect of increasing the flux through the pyruvate dehydrogenase reaction (PDH) on the PHA production. Following this analysis, an increase in the PHA production was predicted in the mutant strain, as the PDH doses were increased. Furthermore, increasing the PDH doses when the strains grow under glucose, an increased flux is observed through ED pathway and gluconeogenesis, with a parallel decreased flux through the OPPP pathway. However, under glycerol growth conditions, an increase of gluconeogenesis is observed with no differences observed in the ED pathway. Finally, as far as it concerns the wild type model, no differences were observed on neither the PHA or PolyP content either the growth rate by an increase of the PDH doses under both conditions. A slight increase was observed in the fluxes towards ED and gluconeogenesis.

Overall, our analysis strongly suggested that, due the blockage of TCA cycle in the *in silico* strain, glucose is metabolized mainly through pentose phosphate (OPPP) instead of Entner-Doudoroff pathway (ED). Therefore, by increasing the flux through PDH reaction ED pathway is favoured increasing the amount of pyruvate, acetyl CoA and subsequently the production of PHA. Taking together, our analysis strongly suggested that the single overexpression of PDH should result in a PHA growth-coupled overproducer strains using sugars (glucose, glycerol) as carbon source.

### Construction of a synthetic operon overexpressing the pyruvate dehydrogenase (BUILD)

Taking advantage of the pMM55 open construct a new plasmid with Golden Gate-MoClo approaches was built, including the overexpression of the pyruvate dehydrogenase complex e.g., *aceF, aceE* and *lpdG.* The resulting plasmid was named pMM62.

For the construction of pMM62, SynPro16 promoter with an upstream λT0 terminator was used in all cases, which was introduced into the Golden Gate-MoClo pL0-PU plasmid (Tiso, T., et al., 2016, Metab. Eng. Commun., 3: 234-244). Genes encoding lpdG (PP_4187), AceE (PP_0339) and AceF (PP_0338) enzymes from *P. putida* KT2440 were cloned intro the pL0-SC plasmid from the Golden Gate-MoClo kit.

Five Golden Gate-MoClo Level 1 reactions were carried out to create transcription unit constructs for the *lpdG, aceE* and *aceF.* In all the transcription units built, the SynPro16 promoter and the corresponding terminators were used. The MoClo system Level 1 plasmids used were pLF-1 (for position 1) to pLF-3 (for position 3), respectively and pLF-6, pLF-7. These positions will allow the correct Level 2 assembly. Correct Level 1 assemblies/plasmids were then mixed with the Level 2 plasmid and the appropriate Level 2 end-linker. The pMM63 level 2 Product vector contained an end-linker L3E for the position 3 that maintained the construct closed, not allowing the addition of extra transcription units if needed.

Correct Level 1 assemblies/plasmids were then mixed with the Level 2 open construct plasmid pMM55, L2 Destination vector and the appropriate Level 2 end-linker. The Level 2 Product vector (pMM62) contained an end-linker L1E for the position 1 that maintained the construct closed, not allowing the addition of extra transcription units if needed. The pMM62 plasmid was then transformed into *P. putida* MM19 and KT2440 Δ*pha* strains by electroporation, yielding MT6 and MT7 strains, respectively.

### Example 3. Results.

Supporting our hypothesis, the removal of the natural PHA cluster, including native regulation, had a positive effect on PHA production. In fact, the strain MT2 harboring genetic iteration 1 and 2, increased wild type PHA production from 4 to 8-fold without appreciable decrease of residual biomass (Table 2). The inclusion of iteration 3, increased even more the PHA production. In fact, MT1 strain more than doubled the PHA production of MT2 strain using aromatic acid as carbon source (Table 2). This increase in PHA production was inversely related with residual biomass, thus suggesting that PHA production was increased at the expense of residual biomass. The accumulative effect on PHA production of the different genetic iterations implemented in *P. putida* becomes evident by using 4HBA as sole carbon source (Table 2). While the wild type strain was not able to produce PHA using this aromatic compound under balanced C/N ration, MT2 (iteration 1 and 2), MT1 (iteration 1-3) and MT6 (iterations 1-4) accumulated, 5.73%, 17.65%, and 21.98% of cellular biomass in form of PHA, respectively.

| **Table 2. Physiological data after 24 h of growth under aromatic substrates (C/N ratio: 1.12 mol/mol)** | | | | | |
|---|---|---|---|---|---|
| Conditions M63 | Strains | Total Biomass (g/L) | PHA (%) | PHA (g/L) | Residual Biomass (g/L) |
| Phenyl acetic acid (4.4 mM) | KT2440 | 0.32 | 0.53 ± 0.10 | 0.00 ± 0.00 | 0.32 ± 0.00 |
| | MT2 | 0.33 | 4.13 ± 0.20 | 0.01 ± 0.00 | 0.31 ± 0.00 |
| | MT1 (72 h) | 0.28 | 14.67 ± 0.10 | 0.04 ± 0.00 | 0.24 ± 0.00 |
| Ferulic acid (3.5 mM) | KT2440 | 0.37 ± 0.01 | 0.80 ± 1.29 | 0.00 ± 0.00 | 0.37 ± 0.01 |
| | MT2 | 0.38 ± 0.00 | 7.78 ± 0.94 | 0.03 ± 0.00 | 0.35 ± 0.00 |
| | MT1 | 0.30 ± 0.00 | 19.68 ± 0.27 | 0.06 ± 0.00 | 0.24 ± 0.00 |
| *p*-Coumaric acid (3.9 mM) | KT2440 | 0.38 ± 0.00 | 0.00 ± 0.00 | 0.00 ± 0.00 | 0.38 ± 0.00 |
| | MT2 | 0.38 ±0.01 | 6.15 ± 0.94 | 0.02 ± 0.00 | 0.36 ± 0.01 |
| | MT1 | 0.24 ± 0.04 | 14.23 ± 1.88 | 0.04 ± 0.01 | 0.21 ± 0.03 |
| Vanillic acid (4.4 mM) | KT2440 (48 h) | 0.27 ± 0.06 | 0.00 ± 0.00 | 0.00 ± 0.00 | 0.27 ± 0.05 |
| | MT2 (48 h) | 0.29 ± 0.03 | 7.68 ± 0.49 | 0.02 ± 0.00 | 0.27 ± 0.02 |
| | MT1 (48 h) | 0.26 ± 0.02 | 15.29 ± 0.33 | 0.04 ± 0.00 | 0.22 ± 0.01 |
| 4-Hydroxybenzoic acid (5 mM) | KT2440 | 0.39 ± 0.02 | 0.00 ± 0.00 | 0.00 ± 0.00 | 0.39 ± 0.01 |
| | KT2440 Δ*pha* | 0.39 ± 0.00 | 0.00 ± 0.00 | 0.00 ± 0.00 | 0.39 ± 0.00 |
| | MT7 | 0.34 ± 0.01 | 13.84 ± 1.38 | 0.05 ± 0.00 | 0.30 ± 0.01 |
| | MT2 | 0.34 ± 0.03 | 5.73 ± 2.16 | 0.02 ± 0.01 | 0.32 ± 0.03 |
| | MT1 | 0.31 ± 0.03 | 17.65 ± 3.30 | 0.05 ± 0.01 | 0.26 ± 0.03 |
| | MT6 | 0.32 ± 0.02 | 21.98 ± 1.84 | 0.07 ± 0.00 | 0.25 ± 0.02 |

Similar pattern was found using glucose and glycerol as carbon source (Table 3). For instance, by using glucose, the production of PHA (in %) of MT2, MT1 and MT6 strains was 14, 27 and 32, respectively. In glycerol, the PHA production for these strains was 10, 30 and 32%.

Interestingly, strain MT7, harboring iterations 1, 2 and 4, despite yielded lower PHA in percentage, accumulated the largest PHA content in absolute terms. This is because MT7 strain yielded the highest amount of total biomass among the engineered strains. Therefore, MT7 stats as a valuable strain for PHA production using sugars as carbon sources.

| **Table 3. Physiological data after 24 h of growth under mono/di saccharides substrates (C/N ratio: 2-2.26 mol/mol)** | | | | | |
|---|---|---|---|---|---|
| Condition M63 | Strains | Total Biomass (g/L) | PHA (%) | PHA (g/L) | Residual Biomass (g/L) |
| Glucose (10 mM) | KT2440 | 0.91 ± 0.02 | 1.57 ± 0.89 | 0.01 ± 0.01 | 0.90 ± 0.01 |
| | KT2440 Δ*pha* | 0.93 ± 0.01 | 0.44 ± 0.25 | 0.00 ± 0.00 | 0.93 ± 0.00 |
| | MT2 | 0.90 ± 0.02 | 14.04 ± 0.73 | 0.12 ± 0.01 | 0.76 ± 0.01 |
| | MT7 | 0.88 ± 0.02 | 28.57 ± 2.13 | 0.25 ± 0.02 | 0.63 ± 0.02 |
| | MT1 | 0.57 ± 0.03 | 27.48 ± 2.45 | 0.15 ± 0.02 | 0.42 ± 0.02 |
| | MT6 | 0.66 ± 0.04 | 32.77 ± 4.49 | 0.22 ± 0.02 | 0.45 ± 0.05 |
| Glucose (1.3 mM) plus Glycerol (20 mM) | KT2440 | 1.02 ± 0.02 | 0.00 ± 0.00 | 0.00 ± 0.00 | 1.02 ± 0.02 |
| | KT2440 Δ*pha* | 0.95 ± 0.03 | 0.00 ± 0.00 | 0.00 ± 0.00 | 0.95 ± 0.03 |
| | MT7 | 0.89 ± 0.07 | 29.73 ± 3.68 | 0.26 ± 0.04 | 0.62 ± 0.04 |
| | MT2 | 0.96 ± 0.04 | 9.73 ± 1.11 | 0.09 ± 0.01 | 0.87 ± 0.02 |
| | MT1 | 0.39 ± 0.05 | 29.98 ± 4.28 | 0.10 ± 0.03 | 0.28 ± 0.01 |
| | MT6 | 0.36 ± 0.09 | 32.20 ± 5.17 | 0.10 ± 0.03 | 0.24 ± 0.07 |

Finally, we decided to validate our strains using both sugars and aromatic, in the presence of small amounts of octanoate as carbon source (Table 4). Confirming previous experiments, large amounts of PHA were achieved even under balanced C/N ratios. Using sugars plus octanoate again MT7 strain stated as the most promising strain yielding up to 0.25 g/l of PHA. However, using aromatics plus octanoate, MT1, MT7 and MT6 shown similar yields (Table 4). Interestingly, using our strain, up to 50% of the total biomass was found in form of PHA.

| **Table 4. Physiological data after 24 h of growth under fatty acid substrate (C/N ratio: 2 - 2.26 mol/mol)** | | | | | |
|---|---|---|---|---|---|
| Conditions M63 | Strains | Total Biomass (g/L) | PHA (%) | PHA (g/L) | Residual Biomass (g/L) |
| Octanoate (1 mM) plus Glycerol (20 mM) | KT2440 | | | | |
| | KT2440 Δ*pha* | 0.99 | 0.44 ± 0.05 | 0.00 ± 0.00 | 0.98 ± 0.00 |
| | MT2 | 0.75 | 13.97 ± 4.07 | 0.11 ± 0.03 | 0.65 ± 0.03 |
| | MT7 | 0.85 ± 0.02 | 29.80 ± 3.76 | 0.25 ± 0.03 | 0.60 ± 0.04 |
| | MT1 | 0.27 | 51.79 ± 3.06 | 0.14 ± 0.01 | 0.13 ± 0.01 |
| | MT6 | 0.24 ± 0.01 | 37.60 ± 2.65 | 0.09 ± 0.01 | 0.15 ± 0.01 |
| Octanoate (3.1 mM) plus4HBA (5 mM) | KT2440 | 0.69 ± 0.05 | 7.09 ± 0.78 | 0.05 ± 0.01 | 0.63 ± 0.05 |
| | KT2440 Δ*pha* | 0.67 ± 0.06 | 0.20 ± 0.23 | 0.00 ± 0.00 | 0.67 ± 0.05 |
| | MT2 | 0.70 ± 0.03 | 26.14 ± 3.72 | 0.18 ± 0.03 | 0.51 ± 0.03 |
| | MT7 | 0.69 ± 0.02 | 41.12 ± 2.03 | 0.28 ± 0.02 | 0.41 ± 0.01 |
| | MT1 | 0.49 ± 0.04 | 46.05 ± 290 | 0.22 ± 0.01 | 0.26 ± 0.03 |
| | MT6 | 0.54 ± 0.01 | 47.94 ± 0.51 | 0.26 ± 0.00 | 0.28 ± 0.01 |

Among the main challenges hampering the optimal production of PHA stand out: i) the mandatory nitrogen limitation required to achieve significant PHA production (which largely limits the amount of bacterial biomass in the bioreactor), and ii) the low carbon flux allocated to synthetize PHA using unrelated carbon sources (which results in negligible PHA production even under Nitrogen limitation). Through 4 model-driven genetic iterations, this invention successfully faces these challenges providing 4 (MT1, MT2, MT6, MT7) bacterial strains able to produce significant amounts of PHA under balanced nutritional conditions even by using unrelated carbon sources.

Iteration 1 and 2 focus on avoiding nitrogen limitation by removing the natural regulation of PHA gene cluster and by building a synthetic operon responsible of constitutive expression of genes required for PHA production. Iteration 3 and 4 are responsible of optimizing the carbon flux towards PHA biosynthesis.

## Claims

1. A recombinant *Pseudomonas putida* KT2440 strain **characterized in that**:
- its *pha* cluster is deleted, and
- it comprises a synthetic operon comprising the nucleotide sequences of the *phaC1* gene, the *phaF* gene, the *phaG* gene and the PP_0763 gene.

2. The recombinant strain according to claim 1, which further comprises deleted the following genes: *mmgF, scpC, sucC, sucD, aceA, glcB, icd, PP_3190*, *ocd, hutF and PP 3533.*

3. The recombinant strain according to any of claims 1 or 2, wherein the synthetic operon further comprises the nucleotide sequences of the *aceF,* the *aceE* and the *lpdG* genes.

4. Use of the recombinant strain according to any one of claims 1 to 3, for the production of polyhydroxyalkanoate.

5. The use according to claim 4, wherein the carbon source used by the strain for the production of polyhydroxyalkanoate is an aromatic compound, sugars, fatty acids, glycerol, a combination of glucose and glycerol, a combination of an aromatic compound and fatty acids, or a combination of sugars and fatty acids.

6. The use according to claim 5, wherein the aromatic compound is selected from the list consisting of: phenyl acetic acid, ferulic acid, *p*-coumaric acid, vanillic acid, 4-hydroxybenzoic acid or any combination thereof.

7. The use according to any of claims 5 or 6, wherein the sugar is glucose.

8. The use according to any one of claims 5 to 7, wherein the fatty acid is octanoate.

9. A method for the production of polyhydroxyalkanoate comprising the following steps:
a. incubating the recombinant strain according to any one of claims 1 to 3 with a carbon source, and
b. recovering the polyhydroxyalkanoate produced after the incubation of step (a).

10. The method according to claim 9, wherein the carbon source is an aromatic compound, sugars, fatty acids, glycerol, a combination of glucose and glycerol, a combination of an aromatic compound and fatty acids, or a combination of sugars and fatty acids.

11. The use according to claim 10, wherein the aromatic compound is selected from the list consisting of: phenyl acetic acid, ferulic acid, *p*-coumaric acid, vanillic acid, 4-hydroxybenzoic acid or any combination thereof.

12. The use according to any of claims 10 or 11, wherein the sugar is glucose.

13. The use according to any one of claims 10 to 12, wherein the fatty acid is octanoate.
